# EUROPÄISCHE PATENTSCHRIFT

(11) **EP 2 323 600 B1**
(45) Veröffentlichungstag und Bekanntmachung des Hinweises auf die Patenterteilung: **26.02.2014**
(21) Anmeldenummer: 09776766.9
(22) Anmeldetag: 18.06.2009
(51) Int. Cl.: A61F 15/00, A61L 2/20, A61F 13/00, A61F 13/02, H05H 1/24

(54) **WUNDSCHNELLVERBAND**
EMERGENCY WOUND DRESSING
PANSEMENT RAPIDE

(30) Priorität: 02.07.2008 DE 102008030913
(43) Veröffentlichungstag der Anmeldung: 25.05.2011
(73) Patentinhaber: Reinhausen Plasma GmbH, 93057 Regensburg (DE)
(72) Erfinder: BISGES, Michael, 93161 Sinzing (DE)
(74) Vertreter: Reichert, Werner Franz
(86) Internationale Anmeldenummer: PCT/EP2009/004395
(87) Internationale Veröffentlichungsnummer: WO 2010/006676

(56) Entgegenhaltungen:
- EP-A- 1 092 404
- WO-A-97/09071
- WO-A-03/099102
- DE-A1- 10 136 403
- DE-A1-102004 049 783
- DE-B3- 10 324 926

## Beschreibung

Die vorliegende Erfindung betrifft einen Wundschnellverband.

Übliche Wundschnellverbände bestehen aus einem Stück Wundauflage, das auf die zu schützende Hautoberfläche, bzw. Wunde aufgebracht wird, wobei die Wundauflage mit einer flexiblen Fixierbahn verbunden ist. Die Wundauflage ist dabei oftmals mit antibakteriell wirkenden Substanzen präpariert und dient dazu, die Wunde schützend abzudecken. Die Fixierbahn erfüllt hingegen die Aufgabe, die Wundauflage aufzunehmen und den Wundschnellverband in einer gewünschten Position zu fixieren.

Für einen erfolgreichen Wundheilungsprozess ist es mit ausschlaggebend, die Wunde vor äußeren bakteriellen Einflüssen zu schützen. Hierfür ist es seit jeher bekannt, Wundschnellverbände zu verwenden und diese vor deren Anwendung zu entkeimen bzw. annähernd steril zu verpacken. Dennoch kann damit nicht verhindert werden, dass sich in dem feuchten Milieu zwischen Wunde und Wundschnellverband Keime ausbilden, die die Wundheilung behindern. Für diesen Fall ist es weiterhin bekannt, beispielsweise jodhaltige Substanzen für die direkte Sterilisation der Wunden zu verwenden.

Neben den jodhaltigen Substanzen gibt es auch entkeimende bzw. sterilisierende Verfahren mittels kalter atmosphärischer Gasentladung. Hier entsteht durch die Gasentladung, bei der Sauerstoff dissoziiert wird, Ozon, das sich auf Grund seiner chemischen Eigenschaften besonders bevorzugt zum Sterilisieren oder Desinfizieren von beispielsweise organischen Materialien eignet, da es eine kurze Halbwertszeit aufweist und sich nach der Behandlung wieder vollständig in Sauerstoff abbaut, ohne dabei toxische Rückstände zu bilden. Die Erzeugung von Ozon erfolgt üblicherweise durch den Betrieb einer mit Hochspannung angeregten dielektrisch behinderten Entladung in einem sauerstoffhaltigen Gas. Dieser Typ von Gasentladung wird aufgrund einer vorhandenen elektrischen Isolierung der Elektroden auch Barriereentladung genannt. Die Barriereentladung spaltet dafür die Sauerstoffmoleküle in den chemisch sehr aktiven atomaren Sauerstoff, welcher sich sofort mit dem nächsten Sauerstoffmolekül zu Ozon (O₃) verbindet. Diese exotherme Reaktion ist sehr schnell und läuft während der Gasentladung ab.

Aufgrund seiner Instabilität kann Ozon jedoch nicht über längere Zeit gelagert oder wie andere industriell verwendete Gase in Druckflaschen gekauft werden. Es ist daher erforderlich, das Ozon vor seiner Anwendung an Ort und Stelle zu erzeugen.

In der Patentschrift DE 103 24 926 B3 wird eine Vorrichtung zur Behandlung organischen Materials mittels einer kalten atmosphärischen Gasentladung offenbart, die als wesentliche Bestandteile eine stiftförmige Elektrode, ein die abgerundete Spitze der Elektrode umgreifendes Dielektrikum und einen Wechselhochspannungsgenerator aufweist, der eine Wechselspannung erzeugt, welche im Betrieb der Vorrichtung an der Elektrode anliegt. Die somit in Längsrichtung, an der Spitze der Elektrode erzeugte Gasentladung bildet bei Anwesenheit von Sauerstoff Ozon, das auf ein von der Spitze der Elektrode beabstandetes biologisches Material abschieden werden kann, so dass beispielweise Mikroorganismen an der Oberfläche des zu behandelnden biologischen Materials gezielt abgetötet werden.

Zwar ist mit der offenbarten Vorrichtung eine Entkeimung oder Sterilisation von biologischem Material, insbesondere einer Wunde möglich, nicht jedoch ein Schutz dieser vor äußeren bakteriellen Einflüssen. Zudem ist der Entkeimungsvorgang relativ umständlich und nur schwer in alltäglichen Situationen einsetzbar, da es zwingend erforderlich ist, die bekannte Vorrichtung mit sich zu tragen und man zudem eine stationäre Energieversorgung benötigt.

Eine Vorrichtung zur Erzeugung von Ozon unter Zuhilfenahme einer elektrischen Entladung ist aus der DE 10 2004 049 783 A1 bekannt. Das Prinzip dieser Vorrichtung besteht im Wesentlichen darin, an der Außenseite der Wand einer abgeschlossenen Aufnahmekammer zwei Elektroden und an der Innenseite dieser gleichen Wand eine Gegenelektrode fest anzuordnen. Die innere Gegenelektrode ist dabei kapazitiv zu den beiden äußeren Elektroden gekoppelt, derart, dass an den beiden äußeren Elektroden eine Wechselspannung anlegbar ist, die an die innere, kapazitiv gekoppelte Gegenelektrode von der äußeren Elektrode lediglich induziert wird. Die innere Gegenelektrode ist isoliert, also gesondert von den äußeren Elektroden und gesondert von einer Spannungsversorgungseinrichtung angeordnet. Insbesondere sind keine elektrischen Zuleitungen zwischen der inneren und den äußeren Elektroden vorgesehen. Mit der bekannten Vorrichtung kann damit eine ausreichend hohe Menge an Ozon über einen ausreichend langen Zeitraum bereitgestellt werden, so dass eine Entkeimung eines beispielsweise temperaturempfindlichen Produktes, eine Desinfektion oder Teilentkeimung von Kosmetika oder Lebensmittel, die sich innerhalb der abgeschlossen Aufnahmekammer befinden, erfolgt.

Mit der bekannten Lösung wird damit eine Vorrichtung vorgeschlagen, die eine ausreichend hohe Menge eines desinfizierenden Gases bereitstellt, jedoch keine Vorrichtung, die es ermöglicht, den Wundheilungsprozess organischen Materials durch Desinfektion zu beschleunigen. Die bekannte Vorrichtung wäre dafür, auf Grund der notwendigen abgeschlossenen Aufnahmekammer in der das zu behandelnde Gut positioniert werden muss, denkbar ungeeignet.

Der Erfindung liegt ausgehend von dem bekannten Stand der Technik die Aufgabe zu Grunde, einen Wundschnellverband bereitzustellen, der eine effiziente Entkeimung, im Speziellen einer Wunde ermöglicht und diese dabei zusätzlich zwischen den Entkeimungsintervallen vor dem Eindringen von Krankheitserregern oder Fremdkörpern schützt. Weiterhin ist es eine Aufgabe der Erfindung, dafür eine einfache und in der Handhabung unkomplizierte Lösung zu bieten, die insbesondere im Alltag Anwendung finden kann.

Diese Aufgabe wird durch einen Wundschnellverband mit den Merkmalen des ersten Patenanspruches gelöst. Die Unteransprüche betreffen besonders bevorzugte Ausgestaltungen der Erfindung.

Der erfindungsgemäße Wundschnellverband sieht dafür eine zusätzliche Elektrodenanordnung vor, bestehend aus zwei äußeren Einkopplungselektroden und einer dazu kapazitiv beabstandeten inneren Gegenelektrode, die mittels der flexiblen Fixierbahn elektrisch zueinander isoliert sind. Die beiden äußeren Einkopplungselektroden sind dabei auf der der Wundauflage abgewandten Seite der Fixierbahn, die innere Gegenelektrode auf der der Wundauflage zugewandten Seite der Fixierbahn angeordnet.

Nach einem weiteren Merkmal der Erfindung ist die kapazitiv gekoppelte Gegenelektrode auf der der Wunde zugewandten Seite von einer gasdurchlässigen Wundauflage umgeben. Damit ist die mindestens eine Gegenelektrode gegenüber dem empfindlichen Bereich der Wunde geschützt. Zudem sorgen Abstandshalter zwischen Wundauflage und Gegenelektrode für einen definierten Abstand und ermöglichen damit eine Gasentladung, die sich in der damit geschaffenen Kammer bevorzugt an den freien Kanten der Gegenelektrode ausbildet.

Nach einer bevorzugten Ausführungsform des erfindungsgemäßen Wundschnellverbandes sind die mindestens zwei Einkopplungselektroden nicht dauerhaft an der Fixierbahn befestigt, sondern bilden eine von dieser lösbare bauliche Einheit mit der Spannungsquelle. Im Zuge der Betriebsicherheit sind die beiden Einkopplungselektroden bei dieser Ausführungsform also fest mit der Spannungsquelle verbunden und von einem zusätzlichen Dielektrikum umschlossen.

Durch die Verwendung eines erfindungsgemäßen Wundschnellverbandes ist es möglich, eine Sterilisation eines biologischen Materials, insbesondere einer Wunde, durchzuführen, ohne dabei den Heilungsprozess durch Entfernen des Wundschnellverbandes zu stören, da dieser während der Reinigung bzw. Entkeimung auf der Wunde verbleiben kann. Weiterhin bietet diese Lösung gegenüber dem Stand der Technik den Vorteil, dass sich durch die Entkeimung der Wunde auch gleichzeitig der Bereich zwischen dem verwendeten Wundschnellverband und der Wunde sterilisiert. Die Funktion eines Wundschnellverbandes wird somit länger und effizienter erfüllt. Im Rahmen der Erfindung sind vielerlei Ausprägungen des Wundschnellverbandes möglich, wie beispielsweise in Stripsform oder in jedweder länglicher Form eines Verbandes. Bei letztgenannter Ausprägung sind eine Vielzahl von Elektrodenanordnungen auf dem Wundschnellverband in einem in Längsrichtung definierten Abstand angebracht, die bei Bedarf stückweise abgetrennt werden können. Dadurch bedingt sind auch verschiedene Ausgestaltungen des verwendeten Dielektrikums möglich und ausdrücklich nicht ausschließlich Textilklebebänder.

Die Erfindung soll nachstehend an Hand von Zeichnungen beispielhaft noch näher erläutert werden.

Es zeigen
- Figur 1: eine schematische Darstellung eines erfindungsgemäßen Wundschnellverbandes zur Sterilisation mittels Gasentladung
- Figur 2: eine schematische Darstellung einer bevorzugten Ausführungsform eines erfindungsgemäßen Wundschnellverbandes
- Figur 3: eine kommerzielle Ausführungsform eines erfindungsgemäßen Wundschnellverbandes in Stripsform.

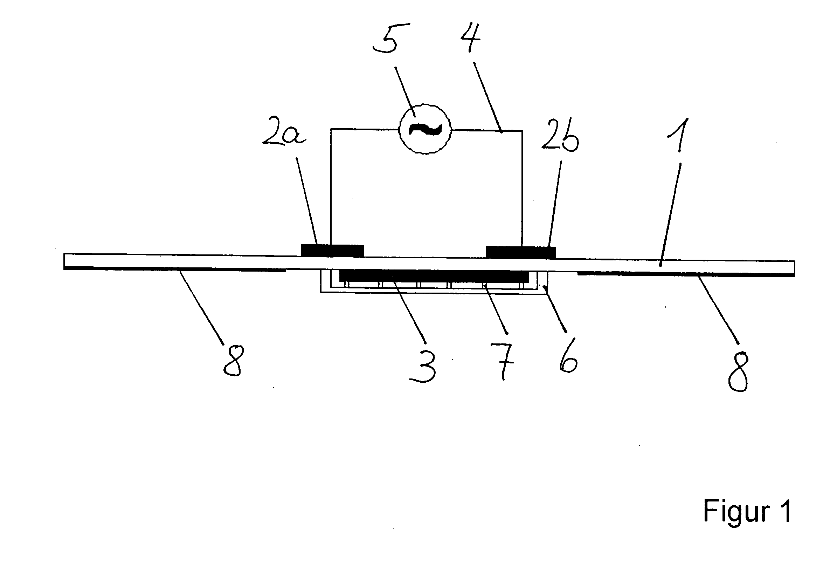

Die Figur 1 zeigt eine schematische Darstellung eines erfindungsgemäßen Wundschnellverbandes, der eine Fixierbahn 1, beispielsweise ein Klebeband oder Textilklebeband, dazu verwendet, um mindestens zwei Einkopplungselektroden 2a und 2b und wenigstens eine dazu kapazitiv gekoppelte Gegenelektrode 3 zueinander elektrisch zu isolieren. Die zwei Einkopplungselektroden 2a und 2b und die Gegenelektrode 3 sind hierfür fest und formschlüssig an der jeweils gegenüberliegenden Seite der Fixierbahn 1 positioniert, derart, dass die mindestens zwei Einkopplungselektroden 2a und 2b an der Außenseite und die wenigstens eine Gegenelektrode 3 an der Innenseite der Fixierbahn 1 befestigt sind. Zudem sind die Einkopplungselektroden 2a und 2b über eine elektrische Leitung 4 mit einer Wechselspannungsquelle 5 verbunden, wobei die Kontaktierung zwischen der elektrischen Leitung 4 und den Einkopplungselektroden 2a und 2b lösbar ist. Da die Wechselspannungsquelle 5 nur im Betrieb an den Einkopplungselektroden 2a und 2b angeschlossen sein muss, kann der erfindungsgemäße Wundschnellverband zwischen den Entkeimungsintervallen wie ein gewöhnlicher Wundschnellverband getragen werden. Die Gegenelektrode 3 ist von einer gasdurchlässigen Wundauflage 6 umgeben, die mittels Abstandshalter 7 von der Gegenelektrode 3 beabstandet gehalten wird. Die Wundauflage 6 steht bei Gebrauch des erfindungsgemäßen Wundschnellverbandes in direktem Kontakt mit der Wunde, die vorliegend aus Übersichtlichkeitsgründen nicht abgebildet ist. Außerdem ist an den Innenseiten des jeweiligen Flügels der Fixierbahn 1 ein Haftmittel 8 vorgesehen, das den Wundschnellverband lösbar mit einer Wunde verbindet. Nach einer anderen, nicht abgebildeten Ausführungsform, kann das Haftmittel 8 auch umlaufend an der Innenseite der Fixierbahn 1 angebracht sein, so dass damit eine verbesserte Haftung des Wundschnellverbandes erreicht wird. Zur Erzeugung einer Gasentladung werden die äußeren Einkopplungselektroden 2a und 2b zeitweise auf hochfrequente Wechselspannung gelegt und diese auf die kapazitiv gekoppelte Gegenelektrode 3 induziert. Dadurch bildet sich bevorzugt entlang der freien Kanten der Gegenelektrode 3 eine elektrische Oberflächenentladung, also eine Plasma nach Art eines Glimmsaumes, aus. Weiterhin wird bei Anwesenheit von Sauerstoff Ozon gebildet, das sich durch die gasdurchlässige Wundauflage 6 auf die Wunde niederschlägt und diese desinfiziert bzw. entkeimt. Damit ist es mit der vorliegenden Erfindung möglich, die Wunde vor äußeren bakteriellen Einflüssen zu schützen und die im feuchten Milieu zwischen Wunde und Wundschnellverband 1 entstandenen Keime wirkungsvoll abzutöten.

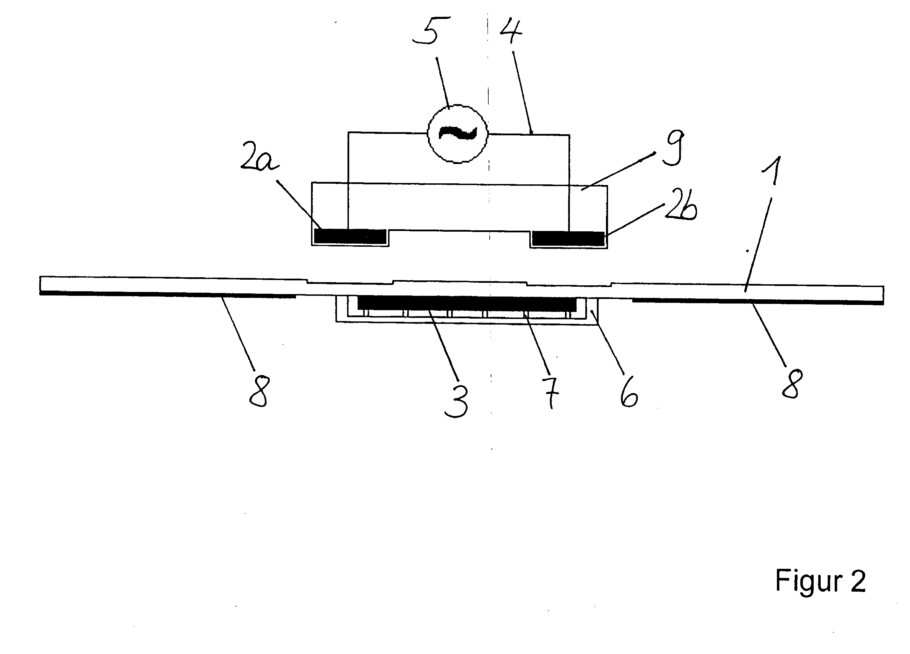

In Figur 2 ist eine bevorzugte Ausführungsform eines erfindungsgemäßen Wundschnellverbandes gezeigt, bei der die beiden Einkopplungselektroden 2a und 2b nicht direkt auf der Fixierbahn 1 angeordnet sind, sondern eine bauliche Einheit mit der Spannungsquelle 5 bilden. Abweichend der Ausführungsform in Figur 1 sind die Einkopplungselektrode 2a und 2b dabei fest mit der elektrischen Leitung 4 verbunden und nicht lösbar. Für einen Entkeimungsvorgang wird die Spannungsquelle 5 samt den fest kontaktierten Einkopplungselektroden 2a und 2b auf der flexiblen Fixierbahn 1, direkt gegenüberliegend der Gegenelektrode 3, positioniert und die eingekoppelte Leistung auf die Gegenelektrode 3 induziert. Zudem sind die Einkopplungselektroden 2a und 2b bei dieser Ausführungsform von einem Dielektrikum 9 umschlossen, was eine ausschließlich kapazitive Wirkungsweise dieser mit der Gegenelektrode 3 gewährleistet.

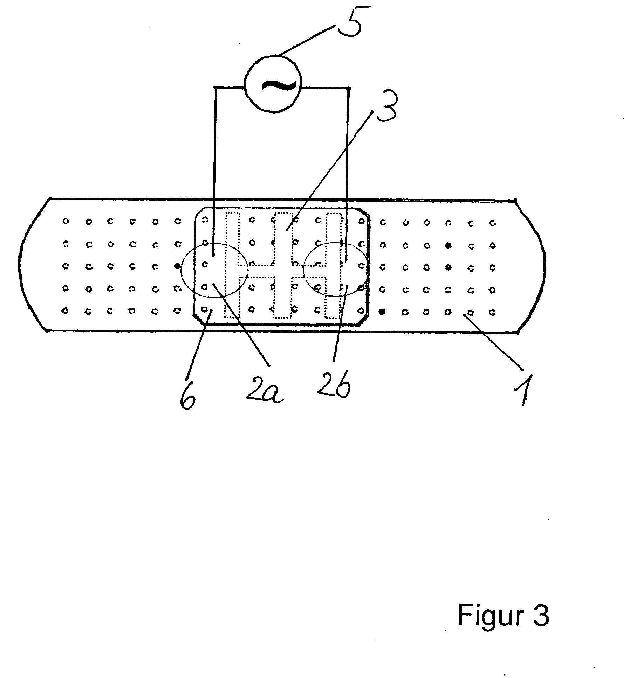

Figur 3 zeigt eine mögliche kommerzielle Ausführungsform des erfindungsgemäßen Wundschnellverbandes in Stripsform nach Figur 1. Die Kontaktierung der abgebildeten Spannungsquelle 5 ist wie bereits geschrieben, lösbar und nur für den intervallmäßigen Entkeimungsbetrieb des erfindungsgemäßen Wundschnellverbandes nötig. Zwischen den Entkeimungsintervallen ist der Wundschnellverband daher wie gewöhnlich zu nutzen.

## Patentansprüche

1. Wundschnellverband,
aufweisend eine Wundauflage (6) und eine Fixierbahn (1),
wobei an der der Wundauflage (6) abgewandten Seite der Fixierbahn (1) mindestens zwei Einkopplungselektroden (2a und 2b) vorgesehen sind, die zumindest zeitweise mit Spannung beaufschlagbar sind
wobei an der der Wundauflage (6) zugewandten Seite der Fixierbahn (1) wenigstens eine Gegenelektrode (3) angeordnet ist,
wobei die Einkopplungselektroden (2a und 2b) und die Gegenelektrode (3) gegenseitig elektrisch isoliert sind,
und wobei die Gegenelektrode (3) von der Wundauflage (6) umgeben ist, **dadurch gekennzeichnet,**
**dass** die Wundauflage (6) zumindest teilweise gasdurchlässig ausgebildet ist, dass die Wundauflage (6) mittels Abstandshalter (7) wenigstens teilweise von der Gegenelektrode (3) beabstandet ist
und **dass** die Gegenelektrode (3) freie Kanten aufweist, derart, dass sich im Falle der zeitweisen Spannungsbeaufschlagung der Einkopplungselektroden (2a und 2b) an den freien Kanten der kapazitiv mit dieser in Wirkverbindung stehenden Gegenelektrode (3) eine elektrische Oberflächenentladung nach Art eines Glimmsaums ausbildet.

2. Wundschnellverband nach Anspruch 1,
**dadurch gekennzeichnet,**
**dass** die mindestens zwei Einkopplungselektroden (2a und 2b) über eine elektrische Leitung (4) mit einer Spannungsquelle (5) kontaktierbar sind.

3. Wundschnellverband nach Anspruch 1 oder 2,
**dadurch gekennzeichnet,**
**dass** der Kontakt zwischen den Einkopplungselektroden (2a und 2b) und der elektrischen Leitung (4) als Dauerkontakt ausgebildet ist.

4. Wundschnellverband nach einem der Ansprüche 1 bis 3,
**dadurch gekennzeichnet,**
**dass** an der der Wundauflage (6) zugewandten Seite der Fixierbahn (1) wenigstens ein Haftbereich (8) vorgesehen ist, der die Wundauflage (6) wenigstens teilweise umschließt.

## Claims

1. Emergency wound dressing, comprising a wound dressing (6) and a fixing web (1),
wherein on the side of the fixing web (1) facing away from the wound dressing (6) at least two coupling electrodes (2a and 2b) are provided, which can be at least intermittently acted upon with voltage,
wherein on the side of the fixing web (1) facing the wound dressing (6) at least one counter-electrode (3) is disposed,
wherein the coupling electrodes (2a and 2b) and the counter-electrode (3) are mutually electrically insulated,
and wherein the counter-electrode (3) is surrounded by the wound dressing (6),
**characterised in**
**that** the wound dressing (6) is configured to be at least partially gas-permeable, that the wound dressing (6) is at least partially spaced apart from the counter-electrode (3) by means of spacers (7),
and **that** the counter-electrode (3) has free edges in such a manner that in the event of the coupling electrodes (2a and 2b) being intermittently acted upon with voltage, a surface discharge in the manner of a transition zone is formed at the free edges of the counter-electrode (3) which is capacitively operatively connected thereto.

2. The emergency would dressing according to claim 1, **characterised in that** the at least two coupling electrodes (2a and 2b) are contactable with a voltage source (5) via an electrical lead (4).

3. The emergency would dressing according to claim 1 or 2, **characterised in that** the contact between the coupling electrodes (2a and 2b) and the electrical lead (4) is configured as a permanent contact.

4. The emergency would dressing according to any one of claims 1 to 3, **characterised in that** on the side of the fixing web (1) facing the wound dressing (6) at least one adhesive region (8) is provided which at least partially encloses the wound dressing (6).

## Revendications

1. Pansement rapide comprenant une compresse (6) et une bande de fixation (1),
dans lequel sont prévues sur la face de la bande de fixation (1) opposée à la compresse (6) au moins deux électrodes de couplage (2a et 2b) qui peuvent être exposées au moins en partie à une tension ;
dans lequel est disposée sur la face de la bande de fixation (1) tournée vers la compresse (6) au moins une contre-électrode (3) ;
dans lequel les électrodes de couplage (2a et 2b) et la contre-électrode (3) sont isolées électriquement les unes des autres ;
et dans lequel la contre-électrode (3) est entourée par la compresse (6), **caractérisé**
**en ce que** la compresse (6) est au moins partiellement perméable aux gaz,
**en ce que** la compresse (6) est tenue au moins partiellement à distance la contre-électrode (3) au moyen d'éléments d'écartement (7),
et **en ce que** la contre-électrode (3) présente des bords libres, de telle façon qu'en cas d'exposition temporaire des électrodes de couplage (2a et 2b) à la tension, il se forme sur les bords libres de la contre-électrode (3) en liaison active capacitive avec celles-ci une décharge superficielle prenant la forme d'une bordure luminescente.

2. Pansement rapide selon la revendication 1, **caractérisé en ce que** les au moins deux électrodes de couplage (2a et 2b) peuvent être mises en contact avec une source de tension (5) par une ligne électrique (4).

3. Pansement rapide selon la revendication 1 ou 2, **caractérisé en ce que** le contact entre les électrodes de couplage (2a et 2b) et la ligne électrique (4) est conçu comme un contact permanent.

4. Pansement rapide selon l'une des revendications 1 à 3, **caractérisé en ce qu'**il est prévu sur la face de la bande de fixation (1) dirigée vers la compresse (6) au moins une zone adhésive (8) qui entoure au moins partiellement la compresse (6).
